# EUROPÄISCHE PATENTSCHRIFT

(11) **EP 0 698 597 B1**
(45) Veröffentlichungstag und Bekanntmachung des Hinweises auf die Patenterteilung: **24.02.1999**
(21) Anmeldenummer: 95112761.2
(22) Anmeldetag: 14.08.1995
(51) Int. Cl.: C07C 229/48, C07D 213/55, A61K 31/215, A61K 31/44, C07C 227/08

(54) **Cyclohexadienderivate**
Cyclohexadiene derivatives
Dérivés de cyclohexadiène

(30) Priorität: 25.08.1994 DE 4430090
(43) Veröffentlichungstag der Anmeldung: 28.02.1996
(73) Patentinhaber: BAYER AG, 51368 Leverkusen (DE)
(72) Erfinder: Urbahns, Klaus, Dr., D-42115 Wuppertal (DE); Heine, Hans-Georg, Dr., D-47800 Krefeld (DE); Junge, Bodo, Dr., D-42399 Wuppertal (DE); Schohe-Loop, Rudolf, Dr., D-42327 Wuppertal (DE); Wollweber, Hartmund, Dr., D-42113 Wuppertal (DE); Sommermeyer, Henning, Dr., D-51061 Köln (DE); Glaser, Thomas, Dr., D-51491 Overath (DE); Wittka, Reilinde, Dr., D-51069 Köln (DE); de Vry, Jean-Marie-Viktor, Dr., D-51503 Rösrath (DE)

(56) Entgegenhaltungen:
- GB-A- 2 012 266
- CHEMICAL AND PHARMACEUTICAL BULLETIN, Bd. 39, Nr. 11, November 1991, TOKYO JP, Seiten 2915-2923, XP000196276 KAZUHIKO TAKE ET AL.: "Agents for treatment of overactive detrusor..."
- EUROPEAN JOURNAL OF MEDICINAL CHEMISTRY, Bd. 27, 1992, PARIS FR, Seiten 527-535, XP000578111 A SAN FELICIANO ET AL.: "Synthesis and pharmacological activities of some pyrido(2,1-b)oxazines"
- ZURNAL ORGANICESKOJ CHIMII, Bd. 30, Nr. 4, 1994, Seiten 528-530, XP000196259 V.V. SOROKIN ET AL.: "Arylaminierung von 2,4-Diacetyl(bis(ethoxycarbonyl))- 5-hydroxy-5-methyl-3-phenyl (2-furyl)-1-cyclohexanonen"
- TETRAHEDRON , Bd. 47, Nr. 32, 1991, OXFORD GB, Seiten 6503-6510, XP002011209 A SAN FELICIANO ET AL.: "2,3,8,8A-Tetrahydro-7H-Oxazolo(3,2-a) -Pyridine: A new heterocyclic system"
- PATENT ABSTRACTS OF JAPAN vol. 014, no. 123 (C-0698), 8.März 1990 & JP-A-02 000129 (FUJISAWA PHARMACEUT CO LTD), 5.Januar 1990,

## Beschreibung

Die vorliegende Erfindung betrifft Cyclohexadienderivate, ein Verfahren zu ihrer Herstellung und ihre Verwendung als Arzneimittel, insbesondere als cerebral wirksame Mittel.

Es ist bereits bekannt, daß 3,6-Cyclohexadien-2-phenyl-1,3-dicarbonsäureester eine Muskelkontraktion inhibierende Wirkung besitzen [vgl. hierzu Chem. Pharm. Bull., 39 (11), 2915 - 23, 1991; GB 87-18906 870810 / GB 87-19441 870817].

GB-A-2 012 266 offenbart 5-Amino-cyclohexa-1,3-dien-3-carbonsäuren und -carbonsäureester als GABA-Transaminaseinhibitoren zur Verwendung gegen Krankheiten des Zentralen Nervensystems. In Zurnal Organiceskoj Chimii 1994, 30, 528 (Russ. J. Org. Chem. 1994, 30, 562) sind die Synthesen von 1-Arylamino-5-phenyl-cyclohexa-1,3-dien-4,6-dicarbonsäureethylestern veröffentlicht. Eur. J. Med. chem. 1992, 27, 527 und Tetrahedr. 1991, 47, 6503 beschreiben 2-(1-Hydroxyprop-3-ylamino)- bzw. 2-(1-Hydroxyeth-2-ylamino)-6-(3-Chlorphenyl)-cyclohexa-1,3-dien-1,5-dicarbonsäuremethylester als Nebenprodukte in der Synthese von Tetrahydropyridinen.

Die Erfindung betrifft Cyclohexadienderivate der allgemeinen Formeln (Ia,b), 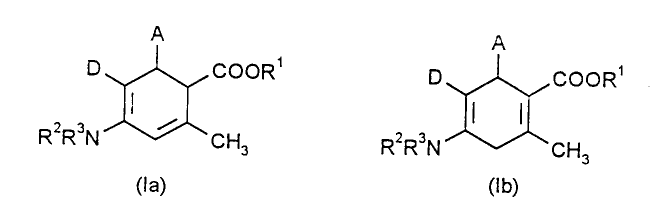 in welcher
- A: für Aryl mit 6 bis 10 Kohlenstoffatomen steht, das gegebenenfalls bis zu 3-fach gleich oder verschieden durch Nitro, Cyano, Cycloalkyl mit 3 bis 7 Kohlenstoffatomen, Halogen, Trifluormethyl oder durch geradkettiges oder verzweigtes Alkylthio, Alkyl oder Alkoxy mit jeweils bis zu 6 Kohlenstoffatomen substituiert ist,
- R¹: für Wasserstoff oder für geradkettiges oder für verzweigtes Alkyl mit bis zu 8 Kohlenstoffatomen steht,
- R² und R³: gleich oder verschieden sind und für Wasserstoff oder für geradkettiges oder verzweigtes Alkyl oder Acyl mit jeweils bis zu 6 Kohlenstoffatomen stehen,
- D: für Nitro oder für geradkettiges oder verzweigtes Alkoxycarbonyl mit bis zu 8 Kohlenstoffatomen steht,
und deren Salze.

Bevorzugte Salze sind physiologisch unbedenkliche Salze. Dies sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (Ia,b),
in welcher
- A: für Phenyl oder Naphthyl steht, die gegebenenfalls bis zu 3-fach gleich oder verschieden durch Nitro, Cyano, Fluor, Chlor, Brom, Iod, Cyclopentyl, Cyclohexyl, Trifluormethyl oder durch geradkettiges oder verzweigtes Alkylthio, Alkyl oder Alkoxy mit jeweils bis zu 4 Kohlenstoffatomen substituiert sind,
- R¹: für Wasserstoff oder für geradkettiges oder verzweigtes Alkyl mit bis zu 6 Kohlenstoffatomen steht,
- R² und R³: gleich oder verschieden sind und für Wasserstoff oder für geradkettiges oder für verzweigtes Alkyl oder Acyl mit jeweils bis zu 4 Kohlenstoffatomen stehen,
- D: für geradkettiges oder verzweigtes Alkoxycarbonyl mit bis zu 6 Kohlenstoffatomen steht,
und deren Salze.

Besonders bevorzugt sind Verbindungen der allgemeinen Formel (Ia,b),
in welcher
- A: für Phenyl steht, das gegebenenfalls bis zu 3-fach gleich oder verschieden durch Nitro, Cyano, Fluor, Chlor, Brom, Iod, Trifluormethyl, Cyclohexyl, Methyl, Methoxy oder durch Methylthio substituiert ist,
- R¹: für Wasserstoff oder für geradkettiges oder verzweigtes Alkyl mit bis zu 4 Kohlenstoffatomen steht,
- R² und R³: gleich oder verschieden sind und für Wasserstoff oder für geradkettiges oder verzweigtes Alkyl oder Acyl mit jeweils bis zu 3 Kohlenstoffatomen stehen
- D: für geradkettiges oder verzweigtes Alkoxycarbonyl mit bis zu 4 Kohlenstoffatomen steht,
und deren Salze.

Außerdem wurde ein Verfahren zur Herstellung der erfindungsgemäßen Verbindungen der allgemeinen Formel (Ia,b) gefunden, dadurch gekennzeichnet, daß man
Verbindungen der allgemeinen Formel (II) 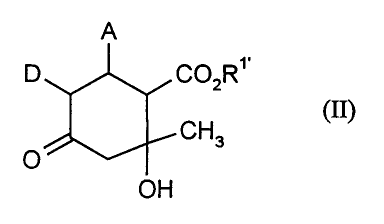 in welcher
A und D die angegebene Bedeutung haben,
   und
R^{1'} die für R¹ angegebene Bedeutung hat, aber nicht für Wasserstoff steht,
   zunächst durch Umsetzung mit Aminen der allgemeinen Formel (III)

   R²R³NH (III)

   in welcher
R² und R³ die oben angegebene Bedeutung haben,
   in inerten Lösemitteln und in Anwesenheit eines Hilfsmittels in die Verbindungen der allgemeinen Formel (IV), 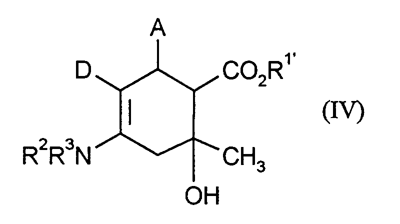 in welcher
A, D, R^{1'}, R² und R³ die oben angegebene Bedeutung haben,
   überführt und in einem zweiten Schritt in einem inerten Lösemittel, gegebenenfalls in Anwesenheit einer Base, und in Anwesenheit eines wasserabscheidenden Hilfsmittels umsetzt,
   und die dabei anfallenden Doppelbindungsisomeren durch Chromatographie und/oder Kristallisation trennt,
   und im Fall R¹ = H, die Ester nach üblichen Methoden hydrolysiert,
   und im Fall R² und/oder R³ ≠ H eine Alkylierung oder Acylierung durchführt.

Das erfindungsgemäße Verfahren kann durch folgendes Formelschema beispielhaft erläutert werden:

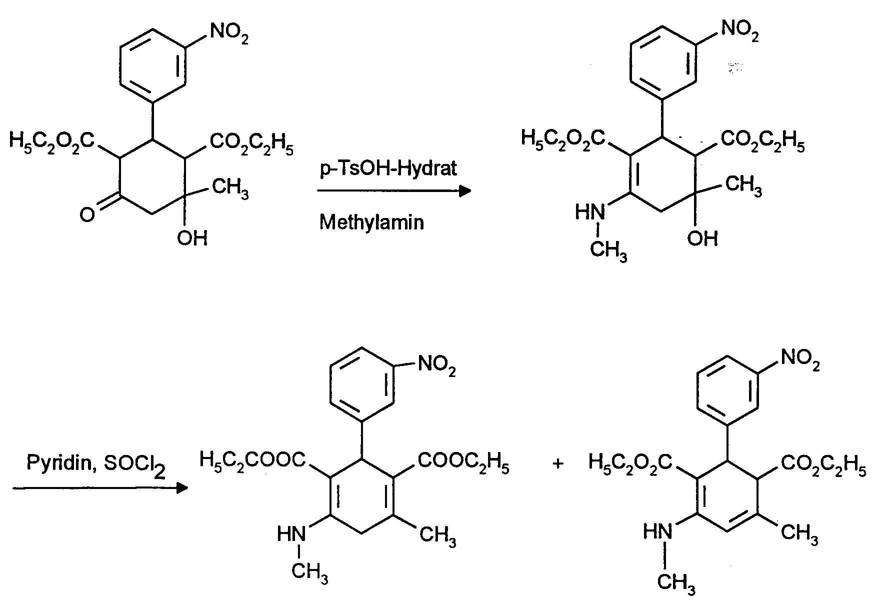

Als Lösemittel für die beiden Verfahrensschritte eignen sich alle inerten organischer Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol oder Toluol, oder Pyridin. Ebenso ist es möglich, Gemische der genannten Lösemittel zu verwenden. Besonders bevorzugt sind Toluol für den ersten Schritt und Pyridin für den zweiten Schritt.

Das Amin wird im allgemeinen in einer Menge von 1 mol bis 5 mol, bevorzugt von 1 mol bis 2 mol, bezogen auf 1 mol der Verbindungen der allgemeinen Formel (II) eingesetzt.

Als Hilfsmittel eignen sich für die Umsetzung der Verbindungen der allgemeinen Formel (II) im allgemeinen organische Sulfonsäuren, wie p-Toluolsulfonsäure oder wasserfreie Mineralsäure wie Phosphorsäure oder Schwefelsäure. Bevorzugt ist p-Toluolsulfonsäure-Hydrat.

Das Hilfsmittel wird in einer Menge von 0,1 mol bis 1 mol, bevorzugt von 0,1 mol bis 0,2 mol jeweils bezogen auf 1 mol der Verbindungen der allgemeinen Formeln (III) und (II) eingesetzt.

Die Umsetzung mit Aminen der allgemeinen Formel (III) erfolgt im allgemeinen in einem Temperaturbereich von 10°C bis 150°C, bevorzugt von 40°C bis 80°C.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Als Hilfsmittel für die Umsetzung mit den Verbindungen der Formel (IV) eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid oder Thionylchlorid, Trifluoressigsäureanhydrid, gegebenenfalls in Anwesenheit von Basen wie Triethylamin, Pyridin oder N-Ethylmorpholin oder N-Methylpiperidin oder Dicyclohexylcarbodiimid und N-Hydroxysuccinimid, Alkoxycarbonylsulfonyltrialkylammoniumhydroxide, Acetanhydrid/NaOAc/Phosphorsäure, Mineralsäuren, wie beispielsweise Schwefelsäure oder organische Sulfonsäuren wie beispielsweise p-Toluolsulfonsäure. Bevorzugt ist Thionylchlorid/Pyridin.

Die Umsetzung der Verbindungen der allgemeinen Formel (IV) erfolgt im allgemeinen in einem Temperaturbereich von 0°C bis 150°C, bevorzugt von 30°C bis 80°C.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Natriumhydrid.

Die Reaktionstemperaturen können in einem größeren Bereich variiert werden. Im allgemeinen arbeitet man zwischen +10°C und +150°C, vorzugsweise zwischen +20°C und +100°C, insbesondere bei Raumtemperatur.

Die Alkylierung wird in den oben aufgeführten Lösemitteln bei Temperaturen von 0°C bis +150° C, vorzugsweise bei Raumtemperaturen bis +100°C durchgeführt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Base wird im allgemeinen in einer Menge von 1 mol bis 5 mol, bevorzugt von 1 mol bis 2 mol jeweils bezogen auf 1 mol der zu alkylierenden Verbindungen eingesetzt.

Als Basen für die Acylierung eignen sich anorganische oder organische Basen. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine, z.B. Trialkyl(C₁-C₆)amine wie Triethylamin, oder Heterocyclen wie Pyridin, Methylpiperidin, Piperidin oder Morpholin. Besonders bevorzugt ist Triethylamin.

Als Lösemittel für die Acylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden oder das jeweilige Acylierungsmittel auch als Lösemittel einzusetzen. Bevorzugt sind Acetanhydrid und Pyridin.

Die Acylierung verläuft im allgemeinen in einem Temperaturbereich von 0°C bis +120°C, vorzugsweise bei +30°C bis +90°C und bei Normaldruck.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Enantiomerenreine Formen erhält man z.B., indem man Diastereomerengemische der Verbindungen der allgemeinen Formel (Ia,b), in welcher R¹ für einen optisch aktiven Esterrest steht, nach üblicher Methode trennt, anschließend entweder direkt umestert oder zuerst die chiralen Carbonsäuren herstellt und dann durch Veresterung die enantiomerenreinen Verbindungen herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Amine der allgemeinen Formel (III) sind bekannt.

Die Verbindungen der allgemeinen Formel (IV) sind bekannt oder können beispielsweise wie oben beschrieben hergestellt werden.

Die Verbindungen der allgemeinen Formel (II) sind bekannt oder können beispielsweise hergestellt werden, indem man

Aldehyde der allgemeinen Formel (V)

A-CHO (V)

in welcher
A die oben angegebene Bedeutung hat,
   mit zwei Equivalenten der Verbindungen der allgemeinen Formel (VI)

   H₃C-CO-CH₂-CO₂R^{1'} (VI)

   in welcher
R^{1'} die oben angegebene Bedeutung hat, in einem organischen Lösemittel und in Anwesenheit einer Base umsetzt.

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Besonders bevorzugt sind Ethanol und Methanol.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Piperidin.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Verbindungen der allgemeinen Formeln (V) und (VI) sind an sich bekannt oder können nach üblichen Methoden hergestellt werden.

Die erfindungsgemäßen Verbindungen der allgemeinen Formel (Ia,b) zeigen ein nicht vorhersehbares, wertvolles pharmakologisches Wirkspektrum.

Sie sind Modulatoren mit Selektivität für Calciumabhängige Kalium-Kanäle großer Leitfähigkeit (BK(Ca)-Kanäle), insbesondere des zentralen Nervensystems.

Aufgrund ihrer pharmakologischen Eigenschaften können sie für die Herstellung von Arzneimitteln zur Behandlung von zentral degenerativen Erkrankungen eingesetzt werden, bei Auftreten von Demenzen wie Multiinfarktdemenz (MID), primär degenerativer Demenz (PDD), präseniler und seniler Demenz vom Typ der Alzheimerschen Krankheit, HIV-Demenz und andere Demenzformen, zur Behandlung der Parkinsonschen Krankheit, amyotrophischer Lateralsklerose sowie multipler Sklerose und Sichelzellenanämie.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Bekämpfung der Folgen cerebraler Durchblutungsstörungen wie cerebraler Ischämien, Schlaganfällen, Schädel-Hirn-Traumen und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüberhinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Bluthochdruck, Arrhythmie, Angina und Diabetes.

### 86Rubidium-Efflux aus C6-BU1-Glioma-Zellen

Die Versuche wurden mit geringfügigen Veränderungen entsprechend der von Tas et al. (Neurosci. Let. 94, 279-284, (1988) beschriebenen Methode durchgeführt. Dazu werden Ratten C6-BU1-Glioma-Zellen verwendet.

Aus den durch Flüssigskeitszintillation genommenen Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Laufmittelgemische:

- a: : Methylenchlorid / AcOEt 10+1
- b: : Methylenchlorid / MeOH 10+1
- c: : PE / AcOEt 7+3
- d: : PE / AcOEt 1+1

### Ausgangsverbindungen

### Beispiel I

### 4-Hydroxy-4-methyl-2-(3-nitrophenyl)-6-oxo-cyclohexan-1,3-dicarbonsäurediethylester

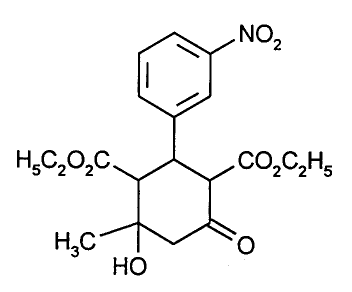

45,3 g (0,3 mol) 3-Nitrobenzaldehyd und 78 g (0,6 mol) Acetessigsäureethylester werden in 300 ml Ethanol gelöst und mit 6 ml Piperidin versetzt. Dann wird 24 h bei 40°C gerührt. Der ausgefallene Feststoff wird abgesaugt und aus Ethanol umkristallisiert. Man erhält 85,4 g der Titelverbindung (Ausbeute 72%).

### Beispiel II

### 6-Hydroxy-6-methyl-4-methylamino-2-(3-nitrophenyl)-cyclohex-3-en-1,3-dicarbonsäurediethylester

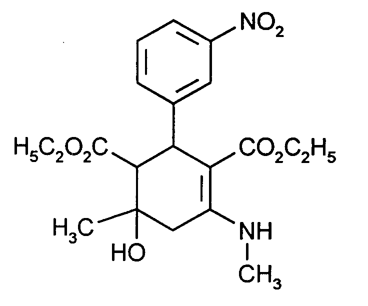

### Variante A:

3,9 g (10 mmol) der Verbindung aus Beispiel I werden in 100 ml Toluol gelöst und mit 0,2 g pTsOH-Hydrat versetzt. Man erhitzt 4 h zum Rückfluß unter wasserabscheidenden Bedingungen und leitet Methylamin ein. Man engt im Vakuum ein und kristallisiert den Rückstand aus Diisopropylether um. Man erhält 400 mg (10% d.Th.) der Titelverbindung.

### Variante B:

19,7 g (50 mmol) der Verbindung aus Beispiel 1 werden in 200 ml Ethanol gelöst und mit 30 ml einer 11 n methanolischen Methylaminlösung sowie 1 g TsOH-Hydrat versetzt. Dann wird 2 h bei 60-65°C gerührt. Nach Einengen der Reaktionsmischung wird der Rückstand an 100 g Kieselgel chromatographisch gereinigt (Methylenchlorid). Das Eluat wird eingeengt und aus Diisopropylether umkristallisiert. Man erhält 17,0 g (84% d.Th.) der Titelverbindung. Smp.: 112°C (Diisopropylether).

### Herstellungsbeispiele

### Beispiel 1

### 4-Methyl-6-methylamino-2-(3-nitrophenyl)cyclohexa-3,6-dien-1,3-dicarbonsäurediethylester

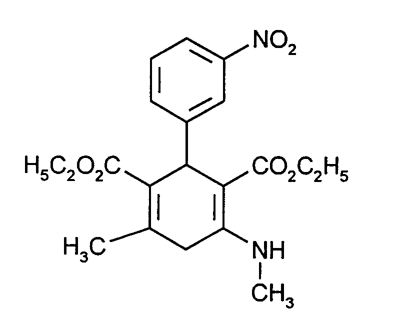

2,5 g (6.2 mmol) der Verbindung aus Beispiel II werden in 30 ml Pyridin vorgelegt, auf 80°C erhitzt und mit 0,95 g (80 mmol) Thionylchlorid versetzt. Man hält 20 min bei dieser Temperatur und erhitzt weitere 20 min zum Rückfluß. Dann engt man ein und nimmt den Rückstand in Methylenchlorid/Wasser auf. Die organische Phase wird abgetrennt, über MgSO₄ getrocknet und eingeengt. Chromatographische Reinigung an Kieselgel (Methylenchlorid : Essigsäureethylester 20+1) und Umkristallisation aus Isopropanol/n-Heptan liefern 0,8 g der Titelverbindung (33% d.Th.)

Die dabei jeweils anfallenden Doppelbindungsisomere werden durch Chromatographie und/oder Kristallisation getrennt. Die angegebenen Ausbeuten beziehen sich auf isolierte Produkte.

### Beispiel 2 und 3

4-Methyl-6-methylamino-2-(4-trifluormethyl-phenyl)cyclohexa-3,6-dien-1,3-dicarbonsäure-dimethylester (2)
6-Methyl-4-methylamino-2-(4-trifluormethyl-phenyl)cyclohexa-3,5-dien-1,3-dicarbonsäure-dimethylester (3)

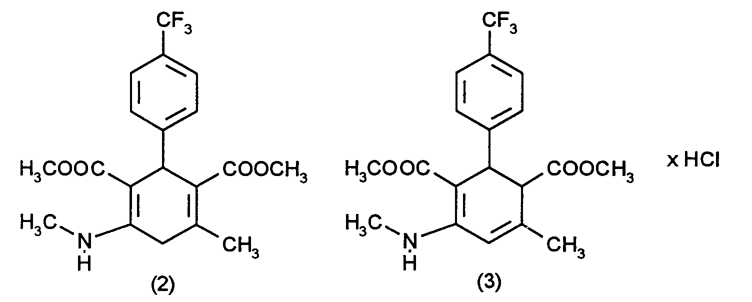

10,0 g (25 mmol) 6-Hydroxy-6-methyl-4-methylamino-2-(4-trifluormethylphenyl)-cyclohex-3-en-1,3-dicarbonsäure-dimethylester (Herstellung analog Beispiel II) werden in 100 ml Pyridin auf 60°C erwärmt und mit 2,5 ml Thionylchlorid versetzt. Man rührt 10 Minuten bei 60°C, engt ein, nimmt den Rückstand in Methylenchlorid auf, wäscht dreimal mit Wasser, trocknet und engt ein. Der Rückstand wird über 200 g Kieselgel grob gereinigt (Petrolether/AcOEt = 3:1) und anschließend durch MPLC aufgetrennt (Methylenchlorid/AcOEt = 30:1). Man erhält zwei Fraktionen. Aus Ether/Petrolether kristallisieren 224 mg (2,3 %) 4-Methyl-6-methylamino-2-(4-trifluormethylphenyl)-cyclohexa-3,6-dien-1,3-dicarbonsäuredimethylester (unpolares Isomer (2)). Aus der 2. Fraktion fällt man aus AcOEt 2,29 g (22 %) des polareren Isomeren, 6-Methyl-4-methylamino-2-(4-trifluormethylphenyl)-cyclohexa-3,5-dien-1,3-dicarbonsäuredimethylester (3) als Hydrochlorid (R_{f} = 0,47 (c)).

In Analogie zu den oben aufgeführten Herstellungsweisen werden die in den Tabellen 1 und 2 genannten Verbindungen hergestellt.

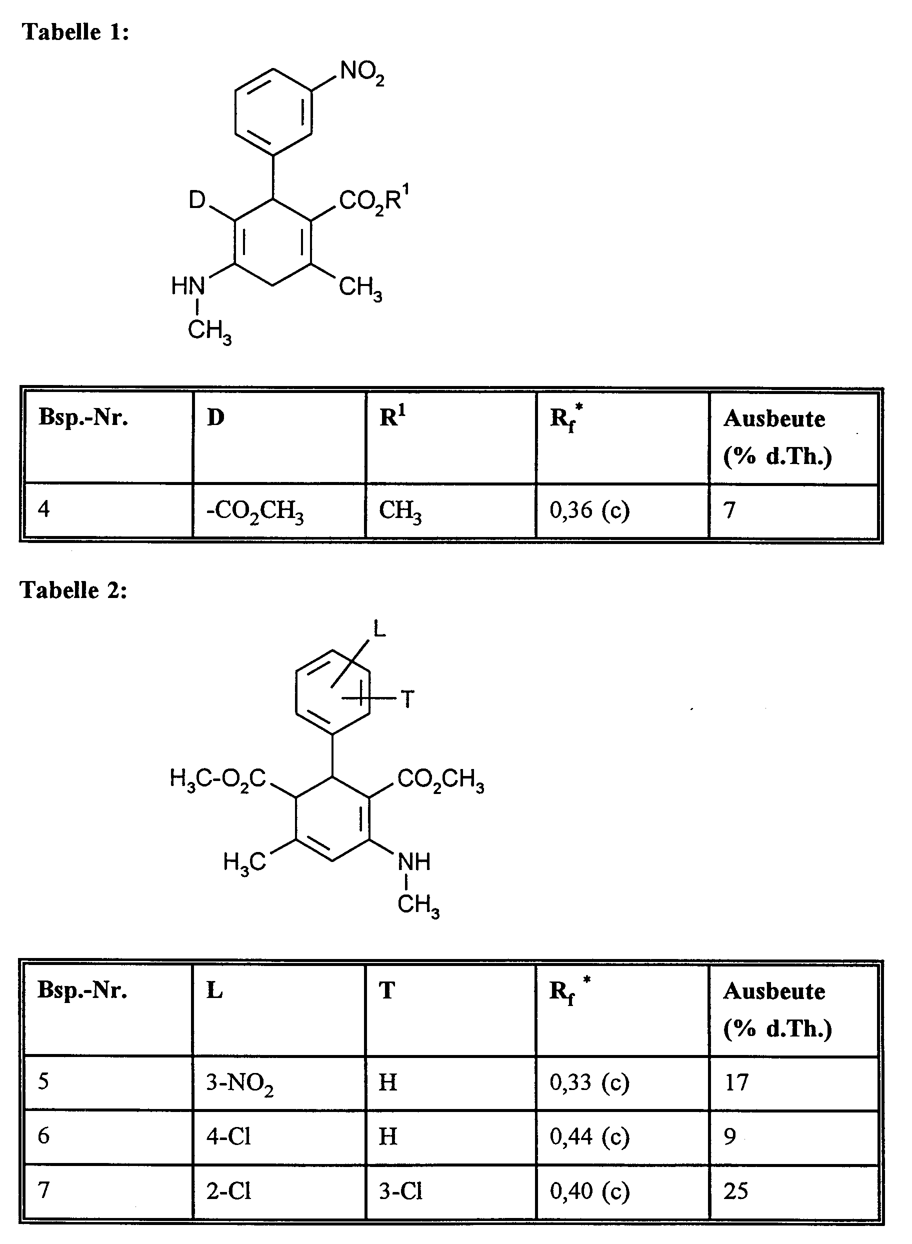

## Patentansprüche

1. Cyclohexadienderivate der allgemeinen Formel (Ia,b) 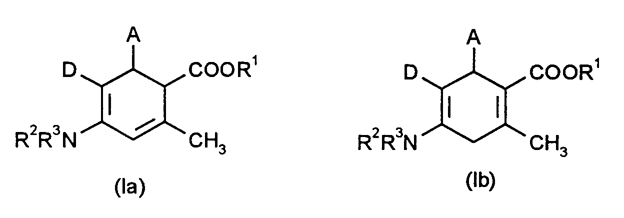 in welcher
A für Aryl mit 6 bis 10 Kohlenstoffatomen oder Pyridyl steht, die gegebenenfalls bis zu 3-fach gleich oder verschieden durch Nitro, Cyano, Halogen, Cycloalkyl mit 3 bis 7 Kohlenstoffatomen, Trifluormethyl oder durch geradkettiges oder verzweigtes Alkylthio, Alkyl oder Alkoxy mit jeweils bis zu 6 Kohlenstoffatomen substituiert sind,
R¹ für Wasserstoff oder für geradkettiges oder für verzweigtes Alkyl mit bis zu 8 Kohlenstoffatomen steht,
R² und R³ gleich oder verschieden sind und für Wasserstoff oder für geradkettiges oder verzweigtes Alkyl oder Acyl mit jeweils bis zu 6 Kohlenstoffatomen stehen,
D für Nitro oder für geradkettiges oder verzweigtes Alkoxycarbonyl mit bis zu 8 Kohlenstoffatomen steht,
und deren Salze.

2. Cyclohexadienderivate nach Anspruch 1,
in welchen
A für Phenyl, Naphthyl oder Pyridyl steht, die gegebenenfalls bis zu 3-fach gleich oder verschieden durch Nitro, Cyano, Fluor, Chlor, Cyclopropyl, Cyclopentyl, Cyclohexyl, Cycloheptyl, Brom, Iod, Trifluormethyl oder durch geradkettiges oder verzweigtes Alkylthio, Alkyl oder Alkoxy mit jeweils bis zu 4 Kohlenstoffatomen substituiert sind,
R¹ für Wasserstoff oder für geradkettiges oder verzweigtes Alkyl mit bis zu 6 Kohlenstoffatomen steht,
R² und R³ gleich oder verschieden sind und für Wasserstoff oder für geradkettiges oder für verzweigtes Alkyl oder Acyl mit jeweils bis zu 4 Kohlenstoffatomen stehen,
D für geradkettiges oder verzweigtes Alkoxycarbonyl mit bis zu 6 Kohlenstoffatomen steht,
und deren Salze.

3. Cyclohexadienderivate nach Anspruch 1 in welchen
A für Phenyl oder Pyridyl steht, die gegebenenfalls bis zu 3-fach gleich oder verschieden durch Nitro, Cyano, Fluor, Chlor, Brom, Cyclohexyl, Iod, Trifluormethyl, Methyl, Methoxy oder durch Methylthio substituiert sind,
R¹ für Wasserstoff oder für geradkettiges oder verzweigtes Alkyl mit bis zu 4 Kohlenstoffatomen steht,
R² und R³ gleich oder verschieden sind und für Wasserstoff oder für geradkettiges oder verzweigtes Alkyl oder Acyl mit jeweils bis zu 3 Kohlenstoffatomen stehen,
D für geradkettiges oder verzweigtes Alkoxycarbonyl mit bis zu 4 Kohlenstoffatomen steht,
und deren Salze.

4. Cyclohexadienderivate nach Anspruch 1 bis 3 zur therapeutischen Anwendung.

5. Verfahren zur Herstellung von Cyclohexadienderivaten nach Anspruch 1 bis 3, dadurch gekennzeichnet, daß man Verbindungen der Formel (II) 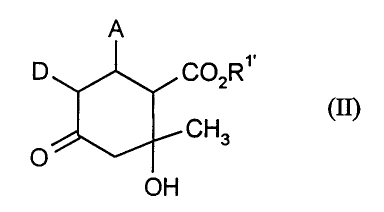 in welcher
A und D die angegebene Bedeutung haben,
und
R^{1'} die für R¹ angegebene Bedeutung hat, aber nicht für Wasserstoff steht,
zunächst durch Umsetzung mit Aminen der allgemeinen Formel (III)
R²R³NH (III)
in welcher
R² und R³ die oben angegebene Bedeutung haben,
in inerten Lösemitteln und in Anwesenheit eines Hilfsmittels in die Verbindungen der allgemeinen Formel (IV), 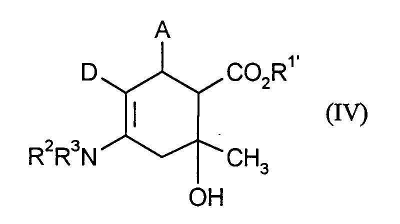 in welcher
A, D, R^{1'}, R² und R³ die oben angegebene Bedeutung haben,
überführt und in einem zweiten Schritt in einem inerten Lösemittel, gegebenenfalls in Anwesenheit einer Base, und in Anwesenheit eines wasserabscheidenden Hilfsmittels umsetzt,
und die dabei anfallenden Doppelbindungsisomeren durch Chromatographie und/oder Kristallisation trennt,
und im Fall R¹ = H, die Ester nach üblichen Methoden hydrolysiert,
und im Fall R² und/oder R³ ≠ H eine Alkylierung oder Acylierung durchführt.

6. Arzneimittel, enthaltend mindestens ein Cyclohexadienderivat nach Anspruch 1 bis 3 oder 5.

7. Arzneimittel nach Anspruch 6 als Modulator mit Selektivität für Calciumabhängige Kalium-Kanäle großer Leitfähigkeit.

8. Verwendung der Cyclohexadienderivate nach Anspruch 1 bis 3 zur Herstellung von Arzneimitteln.

9. Verwendung nach Anspruch 8 zur Herstellung von Modulatoren mit Selektivität für Calcium-abhängige Kalium-Kanäle großer Leitfähigkeit.

## Claims

1. Cyclohexadiene derivatives of the general formula (Ia or b) 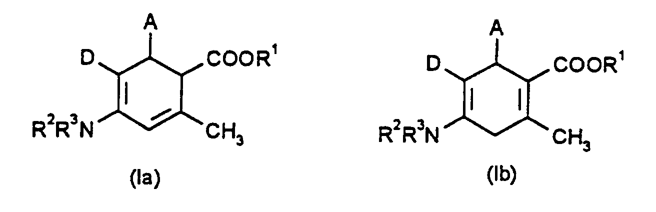 in which
A represents aryl having 6 to 10 carbon atoms or pyridyl, each of which is optionally substituted up to 3 times by identical or different substituents from the group consisting of nitro, cyano, halogen, cycloalkyl having 3 to 7 carbon atoms and trifluoromethyl or straight-chain or branched alkylthio, alkyl or alkoxy in each case having up to 6 carbon atoms,
R¹ represents hydrogen or straight-chain or branched alkyl having up to 8 carbon atoms,
R² and R³ are identical or different and represent hydrogen or straight-chain or branched alkyl or acyl in each case having up to 6 carbon atoms,
D represents nitro or straight-chain or branched alkoxycarbonyl having up to 8 carbon atoms,
and their salts.

2. Cyclohexadiene derivatives according to Claim 1, in which
A represents phenyl, naphthyl or pyridyl, each of which is optionally substituted up to 3 times by identical or different substituents from the group consisting of nitro, cyano, fluorine, chlorine, cyclopropyl, cyclopentyl, cyclohexyl cycloheptyl, bromine, iodine and trifluoromethyl or straight-chain or branched alkylthio, alkyl or alkoxy in each case having up to 4 carbon atoms,
R¹ represents hydrogen or straight-chain or branched alkyl having up to 6 carbon atoms,
R² and R³ are identical or different and represent hydrogen or straight-chain or branched alkyl or acyl in each case having up to 4 carbon atoms,
D represents straight-chain or branched alkoxycarbonyl having up to 6 carbon atoms,
and their salts.

3. Cyclohexadiene derivatives according to Claim 1
in which
A represents phenyl or pyridyl, each of which is optionally substituted up to 3 times by identical or different substituents from the group consisting of nitro, cyano, fluorine, chlorine bromine, cyclohexyl, iodine, trifluoromethyl, methyl and methoxy or methylthio,
R¹ represents hydrogen or straight-chain or branched alkyl having up to 4 carbon atoms,
R² and R³ are identical or different and represent hydrogen or straight-chain or branched alkyl or acyl in each case having up to 3 carbon atoms,
D represents straight-chain or branched alkoxycarbonyl having up to 4 carbon atoms,
and their salts.

4. Cyclohexadiene derivatives according to Claims 1 to 3 for therapeutic use.

5. Process for the preparation of cyclohexadiene derivatives according to Claims 1 to 3, characterized in that compounds of the formula (II) 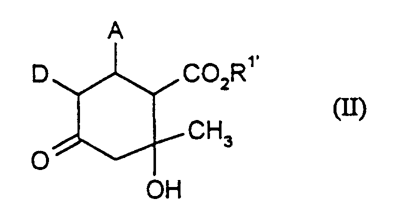 in which
A and D have the meaning specified,
and
R^{1'} has the meaning specified for R¹, but does not represent hydrogen,
are first converted by reaction with amines of the general formula (III)
R²R³NH (III)
in which
R² and R³ have the meaning specified above,
in inert solvents and in the presence of an auxiliary into the compounds of the general formula (IV) 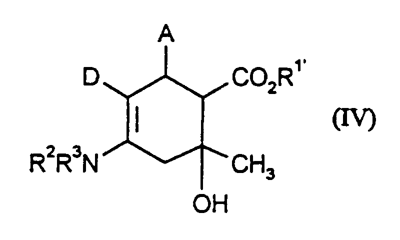 in which
A, D, R^{1'}, R² and R³ have the meaning specified above,
and in a second step reacted in an inert solvent, if appropriate in the presence of a base, and in the presence of a dehydrating auxiliary,
and the double bond isomers obtained in this process are separated by chromatography and/or crystallization
and if R¹ = H, the esters are hydrolysed by customary methods,
and if R² and/or R³ ≠ H, an alkylation or acylation is carried out.

6. Medicaments, containing at least one cyclohexadiene derivative according to Claims 1 to 3 or 5.

7. Medicaments according to Claim 6 as modulators having selectivity for calcium-dependent potassium channels of high conductivity.

8. Use of the cyclohexadiene derivatives according to Claims 1 to 3 for the production of medicaments.

9. Use according to Claim 8 for the production of modulators having selectivity for calcium-dependent potassium channels of high conductivity.

## Revendications

1. Dérivés de cyclohexadiène de formule générale (Ia, b) : 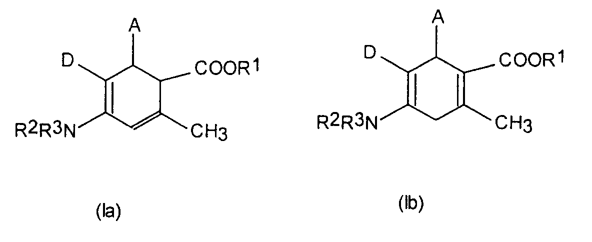 dans lesquelles :
A représente aryle ayant 6 à 10 atomes de carbone ou pyridyle, qui sont facultativement jusqu'à 3 fois substitués, de manière identique ou différente, par nitro, cyano, halogène, cycloalcoyle ayant 3 à 7 atomes de carbone, trifluorométhyle ou par alcoylthio, alcoyle ou alcoxy linéaire ou ramifié, ayant chaque fois, jusqu'à 6 atomes de carbone ;
R¹ représente hydrogène ou alcoyle linéaire ou ramifié ayant jusqu'à 8 atomes de carbone ;
R² et R³ sont identiques ou différents et représentent hydrogène ou alcoyle ou acyle linéaire ou ramifié ayant chaque fois, jusqu'à 6 atomes de carbone ;
D représente nitro ou alcoxycarbonyle linéaire ou ramifié ayant jusqu'à 8 atomes de carbone,
et leurs sels.

2. Dérivés de cyclohexadiène suivant la revendication 1, dans lesquels :
A représente phényle, naphtyle ou pyridyle, qui sont facultativement jusqu'à 3 fois substitués, de manière identique ou différente, par nitro, cyano, fluor, chlore, cyclopropyle, cyclopentyle, cyclohexyle, cycloheptyle, brome, iode, trifluorométhyle, ou par alcoylthio, alcoyle ou alcoxy linéaire ou ramifié, ayant chaque fois, jusqu'à 4 atomes de carbone ;
R¹ représente hydrogène ou alcoyle linéaire ou ramifié ayant jusqu'à 6 atomes de carbone ;
R² et R³ sont identiques ou différents et représentent hydrogène ou alcoyle ou acyle linéaire ou ramifié ayant chaque fois, jusqu'à 4 atomes de carbone ;
D représente alcoxycarbonyle linéaire ou ramifié ayant jusqu'à 6 atomes de carbone,
et leurs sels.

3. Dérivés de cyclohexadiène suivant la revendication 1, dans lesquels :
A représente phényle ou pyridyle, qui sont facultativement jusqu'à 3 fois substitués, de manière identique ou différente, par nitro, cyano, fluor, chlore, brome, cyclohexyle, iode, trifluorométhyle, méthyle, méthoxy ou méthylthio ;
R¹ représente hydrogène ou alcoyle linéaire ou ramifié ayant jusqu'à 4 atomes de carbone ;
R² et R³ sont identiques ou différents et représentent hydrogène ou alcoyle ou acyle linéaire ou ramifié ayant chaque fois, jusqu'à 3 atomes de carbone ;
D représente alcoxycarbonyle linéaire ou ramifié ayant jusqu'à 4 atomes de carbone,
et leurs sels.

4. Dérivés de cyclohexadiène suivant les revendications 1 à 3, pour une utilisation thérapeutique.

5. Procédé de préparation des dérivés de cyclohexadiène suivant les revendications 1 à 3, caractérisé en ce que l'on transforme
des composés de formule (II) : 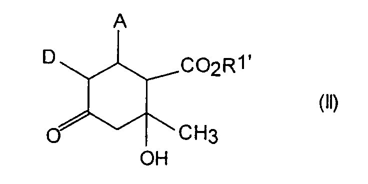 dans laquelle :
A et D ont les significations données, et
R^{1'} a la signification donnée pour R¹, mais ne représente pas hydrogène,
d'abord par réaction avec des amines de formule générale (III) :
R²R³NH (III)
dans laquelle
R² et R³ ont les significations données ci-dessus,
dans un solvant inerte et en présence d'un adjuvant, en les composés de formule générale (IV) : 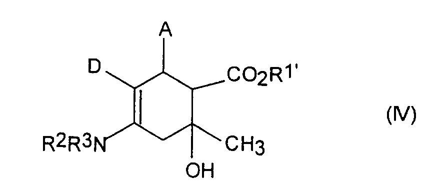 dans laquelle
A, D, R^{1'}, R² et R³ ont les significations données ci-dessus,
et dans une deuxième étape, on fait réagir dans un solvant inerte, facultativement en présence d'une base, et en présence d'un adjuvant déshydratant,
et l'on sépare les isomères de la double liaison ainsi formés, par chromatographie et/ou cristallisation,
et dans le cas où R¹ = H, l'ester est hydrolysé par des procédés connus,
et dans le cas où R² et/ou R³ ≠ H, on réalise une alcoylation ou une acylation.

6. Agent pharmaceutique contenant au moins un dérivé de cyclohexadiène suivant les revendications 1 à 3 ou 5.

7. Agent pharmaceutique suivant la revendication 6, comme modulateur pour une plus grande conductibilité avec sélectivité pour le canal potassique dépendant du calcium.

8. Utilisation des dérivés de cyclohexadiène suivant les revendications 1 à 3, pour préparer un agent pharmaceutique.

9. Utilisation suivant la revendication 8, pour préparer des modulateurs pour une plus grande conductibilité avec sélectivité pour le canal potassique dépendant du calcium.
